# EUROPEAN PATENT APPLICATION

(11) **EP 4 116 712 A1**
(43) Date of publication of application: **11.01.2023**
(21) Application number: 21775071.0
(22) Date of filing: 08.03.2021
(51) Int. Cl.: G01N 33/543

(54) **TEST STRIP**

(30) Priority: 23.03.2020 JP 2020050654
(71) Applicant: Terumo Kabushiki Kaisha, Tokyo 151-0072 (JP)
(72) Inventor: MORIUCHI, Takeyuki, Nakakoma-gun, Yamanashi 409-3853 (JP); HAYASHIDA, Yuma, Nakakoma-gun, Yamanashi 409-3853 (JP)
(74) Representative: Casalonga
(86) International application number: PCT/JP2021/008925
(87) International publication number: WO 2021/192952

(57) **Abstract**

A test strip (12) includes a flow path (26) formed in a main body portion (20); a reagent portion (22b) provided in the flow path (26); and an intake portion (24) which is provided at a starting end of the flow path (26) and through which a sample is introduced into the flow path (26). The main body portion (20) is provided with a buffer space (28) communicating with a terminal end of the flow path (26), and a vent hole (30) opened at an outer surface of the main body portion (20) and communicating with the buffer space (28), and in a region where the buffer space (28) and the flow path (26) are spatially connected, a cross-sectional area (Sb) of the buffer space (28) is larger than a cross-sectional area (S) of the flow path (26).

## Description

### Technical Field

The present invention relates to a test strip.

### Background Art

For example, JP-A-2007-10558 discloses a test strip including a main body portion provided with a flow path through which blood (a sample) flows, a reagent portion disposed in the flow path, a vent hole for discharging air in the flow path, and a volume swelling member provided in the vent hole. When the blood is guided to a terminal end of the flow path, the volume swelling member expands by the blood and closes the vent hole. Accordingly, the blood flowing in the flow path can be inhibited from leaking to an outside of the main body portion.

### Summary of Invention

In a test strip such as the test strip as described in JP-A-2007-10558, it is necessary to provide a volume swelling member, and thus costs of the test strip may increase.

The invention has been developed in view of such a problem, and an object thereof is to provide a test strip capable of preventing a sample in a flow path from leaking from a vent hole to an outside of a main body portion while avoiding an increase in costs.

According to one aspect of the invention provides a test strip including: a flow path formed in a main body portion; a reagent portion provided in the flow path; and an intake portion which is provided at a starting end of the flow path and through which a sample is introduced into the flow path, in which the main body portion is provided with a buffer space communicating with a terminal end of the flow path, and a vent hole opened at an outer surface of the main body portion and communicating with the buffer space, and in a region where the buffer space and the flow path are spatially connected, a cross-sectional area of the buffer space is larger than a cross-sectional area of the flow path.

According to the invention, in the region where the buffer space and the flow path are spatially connected, the cross-sectional area of the buffer space is larger than the cross-sectional area of the flow path. Therefore, at a connection portion between the buffer space and the flow path, which is the terminal end of the flow path, an interfacial tension component in a flow direction is reduced for blood in the flow path. Accordingly, a capillary force that causes the blood to be drawn into the buffer space is less likely to occur. Even if the sample in the flow path flows into the buffer space, the sample can be kept in the buffer space. Accordingly, the sample can be inhibited from leaking from the vent hole to the outside of the main body portion while avoiding an increase in costs.

### Brief Description of Drawings

[FIG. 1] FIG. 1 is a plan view illustrating an overall configuration of a component measurement system including a test strip according to an embodiment of the invention.
[FIG. 2] FIG. 2 is a perspective view of the test strip in FIG. 1.
[FIG. 3] FIG. 3 is an exploded perspective view of the test strip in FIG. 2.
[FIG. 4] FIG. 4 is a vertical cross-sectional view of the test strip in FIG. 2.
[FIG. 5] FIG. 5A is a cross-sectional view taken along a line VA-VA of FIG. 4, and FIG. 5B is a cross-sectional view taken along a line VB-VB of FIG. 4.
[FIG. 6] FIG. 6 is a plan view of the test strip in FIG. 2 viewed from one side in a thickness direction.
[FIG. 7] FIG. 7 is a flowchart illustrating a manufacturing process of the test strip in FIG. 2.
[FIG. 8] FIG. 8 is a cross-sectional illustration view for illustrating a displacement amount of the test strip.
[FIG. 9] FIG. 9 is a partially omitted cross-sectional view of the component measurement system in FIG. 1.
[FIG. 10] FIG. 10A is a vertical cross-sectional view of a test strip according to a first embodiment, and FIG. 10B is a plan view of the test strip in FIG. 10A as viewed from a thickness direction.
[FIG. 11] FIG. 11A is a vertical cross-sectional view of a test strip according to Comparative Example 1, and FIG. 11B is a plan view of the test strip in FIG. 11A as viewed from a thickness direction.
[FIG. 12] FIG. 12A is a vertical cross-sectional view of a test strip according to Comparative Example 2, and FIG. 12B is a plan view of the test strip in FIG. 12A as viewed from a thickness direction.
[FIG. 13] FIG. 13A is a vertical cross-sectional view of a test strip according to Comparative Example 3, and FIG. 13B is a plan view of the test strip in FIG. 13A as viewed from a thickness direction.
[FIG. 14] FIG. 14A is a vertical cross-sectional view of a test strip according to Comparative Example 4, and FIG. 14B is a plan view of the test strip in FIG. 14A from a thickness direction.
[FIG. 15] FIG. 15 is a table illustrating a first test result.
[FIG. 16] FIG. 16 is a graph illustrating a second test result.

### Description of Embodiments

Hereinafter, preferred embodiments of a test strip according to the invention will be described with reference to the accompanying drawings.

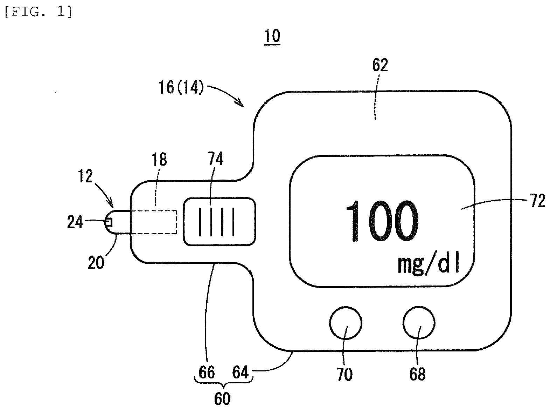

As illustrated in FIG. 1, a component measurement system 10 according to an embodiment of the invention includes a test strip 12 capable of holding a sample, and a component measurement device 14 for measuring an amount of an analyte contained in the sample by attaching the test strip 12.

A sample is introduced into the test strip 12. The test strip 12 is configured to be held at a detection target position in the component measurement device 14 in a state (color-developing state) in which the sample reacts with a reagent to develop a color inside the test strip 12. Meanwhile, the component measurement device 14 optically detects a reaction product of the sample and the reagent at the detection target position of the test strip 12. The test strip 12 may also be referred to as a chip, a sensor, or the like. The "sample" may be whole blood (blood) or may be separated blood plasma. The sample may be another body fluid or an aqueous solution containing analytes.

Hereinafter, the component measurement system 10 (blood glucose level measurement system) that detects the amount of an analyte (here, glucose) when the sample is blood will be representatively described. In particular, the component measurement device 14 is configured as a blood glucose meter 16 that performs blood glucose level measurement by including a measurement unit 18 that irradiates the detection target position with measurement light having a predetermined wavelength and detects measurement light (transmitted light) transmitted through a detection target.

The test strip 12 includes a reagent. The reagent contains a color-developing reagent that dissolves in the sample and reacts according to the amount of an analyte in the sample. Therefore, when the reagent and the analyte come into contact with each other, a color-developing reaction occurs in which the color-developing reagent develops a color, and a color-developing component (reaction product) is generated. The reagent according to the present embodiment reacts specifically with glucose. Examples of the reagent according to the present embodiment include a mixed reagent of (i) glucose oxidase (GOD), (ii) peroxidase (POD), (iii) 1-(4-sulfophenyl)-2,3-dimethyl-4-amino-5-pyrazolone, (iv) N-ethyl-N-(2-hydroxy-3-sulfopropyl)-3,5-dimethylaniline, sodium salts, and monohydrates (MAOS), or a mixed reagent of glucose dehydrogenase (GDH) and tetrazolium salts. The reagent may contain a buffer such as a phosphate buffer, a mediator, and an additive. A type and a component of the reagent are not limited to these. In the present embodiment, the blood glucose meter 16 detects a mixture of the color-developing component (reaction product) and the sample. In particular, when the measurement light having a predetermined wavelength is emitted to the detection target position and the measurement light (transmitted light) transmitted through the detection target is detected, a prepared mixed reagent solution is preferably applied directly to a predetermined position in the test strip 12 and dried without using a porous member or a carrier.

The component measurement system 10 is used as a measurement system for personal use, which is operated by a user (patient). For example, the user uses the test strip 12 and the blood glucose meter 16 to measure a blood glucose level and manage his/her own blood glucose. The component measurement system 10 may be used in a medical facility or the like as a device for measuring the blood glucose level of a patient by a health care worker.

When the test strip 12 is attached to the blood glucose meter 16, a part of the test strip 12 protrudes outward of the blood glucose meter 16. The test strip 12 includes an opening portion (an intake portion 24) in the part protruding from the blood glucose meter 16. The blood glucose level measurement is executed by the blood glucose meter 16 by introducing the blood into the test strip 12 via the intake portion 24. The test strip 12 is a disposable device that is discarded after each measurement.

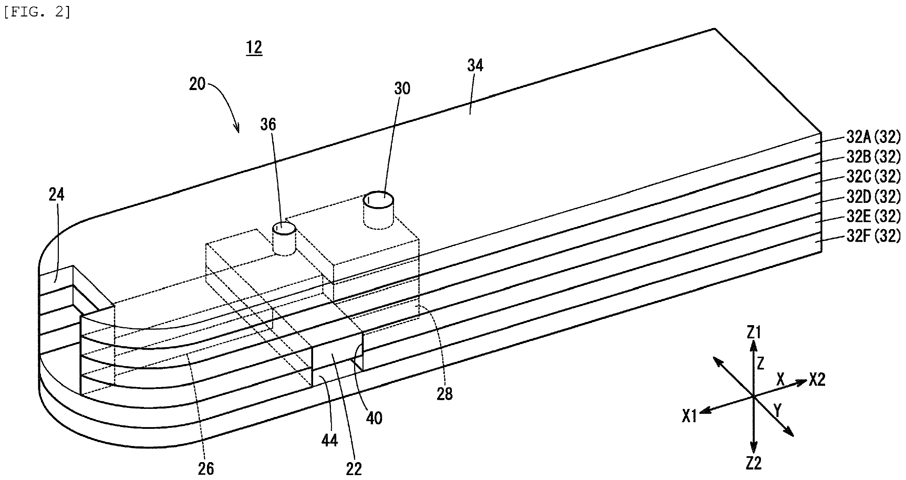

As illustrated in FIG. 2, the test strip 12 includes a test paper-shaped (flat plate-shaped) main body portion 20 and a reagent piece 22 (reagent member) provided on the main body portion 20. A direction in which the main body portion 20 is inserted into or removed from the blood glucose meter 16 is a long axis direction (an arrow X direction) of the main body portion 20. Here, when the main body portion 20 is attached to the blood glucose meter 16, one end (an arrow X1 direction) of the main body portion 20 is exposed from the blood glucose meter 16, and the other end (an arrow X2 direction) of the main body portion 20 is accommodated in the blood glucose meter 16. One end portion (an end portion in the arrow X1 direction) of the main body portion 20 is formed in a substantially semicircular shape when viewed from a thickness direction of the main body portion 20 (viewed from an arrow Z direction). The other end portion (an end portion in the arrow X2 direction) of the main body portion 20 is formed in a rectangular shape when viewed from the thickness direction of the main body portion 20 (viewed from the arrow Z direction). That is, an outer shape of the main body portion 20 is a substantially rectangular shape with one side bulging in an arc shape when viewed from the thickness direction. When the main body portion 20 is attached to the blood glucose meter 16, a region from the other end (the end portion in the arrow X2 direction) of the main body portion 20 to at least the reagent piece 22 is accommodated in the blood glucose meter 16.

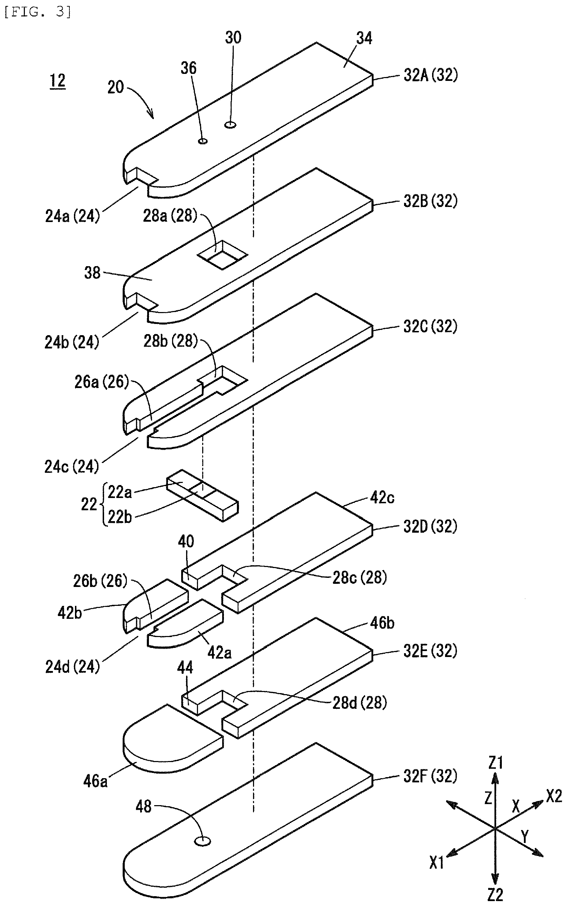

As illustrated in FIGS. 2 to 4, the main body portion 20 is formed by stacking and integrating a plurality of plate bodies 32 in a thickness direction (the arrow Z direction) of the plate bodies 32. Hereinafter, the plurality of plate bodies 32 are referred to as a first plate body 32A, a second plate body 32B, a third plate body 32C, a fourth plate body 32D, a fifth plate body 32E, and a sixth plate body 32F from an upper direction (an arrow Z1 direction) toward a lower direction (an arrow Z2 direction) in FIG. 3. Outer edges of the first to sixth plate bodies 32A to 32F are formed in substantially the same shape in a plan view from the arrow Z direction. More specifically, the outer edges of the plurality of plate bodies 32 are formed in a substantially rectangular shape having an arc at one end portion in the plan view from the arrow Z direction. In addition, space portions such as cutout portions 24a to 24d and a vent hole 30 to be described later are appropriately cut out in the plurality of plate bodies 32. An adhesive layer (not illustrated) made of an adhesive or the like is provided between the plate bodies 32 adjacent to each other. The adjacent plate bodies 32 are firmly adhered to each other by the adhesive layer.

The main body portion 20 is provided with the intake portion 24 for introducing the blood into the main body portion 20, a flow path 26 for guiding the blood introduced into the intake portion 24 to the reagent piece 22, a buffer space 28 for communicating with the flow path 26, and the vent hole 30 for communicating with the buffer space 28. The intake portion 24 is provided on one end (the arrow X1 direction) of the main body portion 20 formed in an arc shape in the plan view in the arrow Z direction. The blood is transferred by capillary force through the flow path 26. One surface of the intake portion 24 in the arrow Z1 direction is opened, and the other surface of the intake portion 24 in the arrow Z2 direction is covered by the fifth plate body 32E. In this case, the surface of the intake portion 24 in the arrow Z2 direction may be covered by the sixth plate body 32F instead of the fifth plate body 32E, or may be covered by the fifth plate body 32E and the sixth plate body 32F. One end (the arrow X1 direction) of the flow path 26 is opened in the intake portion 24. A length of the intake portion 24 in a width direction is larger than a length of the flow path 26 in the width direction. The intake portion 24, the flow path 26, and the buffer space 28 are formed by stacking the space portions formed in the respective plate bodies 32.

The plurality of plate bodies 32 may be made of a resin material such as polyethylene terephthalate (PET), polymethyl methacrylate (PMMA), polyesters, polycarbonates, polystyrenes, polypropylenes, an acrylonitrile-butadienestyrene copolymer (ABS), a cycloolefin polymer (COP), or a cyclic olefin copolymer (COC). When the component measurement device 14 is of a type that detects the measurement light (transmitted light) transmitted through the detection target, a path of the measurement light is made of a transparent material. The plate bodies 32 may be mixed with a pigment according to a purpose, and when the plate bodies 32 are a light shielding member, a resin material containing carbon black is used. A light shielding rate of the light shielding member is preferably 90% or higher based on a measurement method of JIS K7605; 1976 (obsolete standard), and a black film member having a light shielding rate of 99% or higher can be preferably used. A thickness of each of the plate bodies 32 is preferably 20 µm to 150 µm, and more preferably 20 µm to 100 µm.

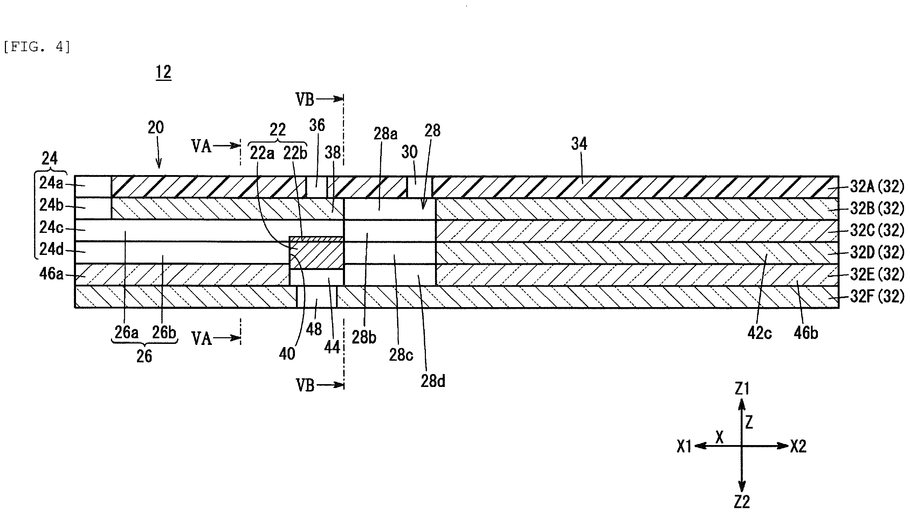

As illustrated in FIGS. 3 and 4, the first plate body 32A is a planar member disposed at one end of the test strip 12 in the thickness direction (the end in the arrow Z1 direction). The first plate body 32A is provided with the first cutout portion 24a and the vent hole 30.

The first cutout portion 24a forms a part of the intake portion 24. The first cutout portion 24a is formed at an end portion of the first plate body 32A in the arrow X1 direction. The first cutout portion 24a is formed in a rectangular shape with one side opened in the plan view from the arrow Z direction.

A light shielding black film member may be used as the first plate body 32A. When the light shielding member is used as the first plate body 32A, a first opening portion 36 (aperture) is further formed on the first plate body 32A. Accordingly, the first plate body 32A constitutes a light shielding portion 34 in the test strip 12, which shields a part of the measurement light. The first opening portion 36 is provided independently at a position away from the first cutout portion 24a by a predetermined distance in the arrow X2 direction. The first opening portion 36 is a through-hole of the first plate body 32A through which the measurement light passes in the thickness direction of the test strip 12. The first opening portion 36 is located substantially at a center of the first plate body 32A in the width direction (an arrow Y direction). The first opening portion 36 is formed in a circular shape. The first plate body 32A may be provided with a transparent portion (a light guiding portion) capable of transmitting the measurement light in place of the first opening portion 36. The first opening portion 36 allows the measurement light in an amount required for the optical detection of the reaction product (a measurement target) of the sample and the reagent to reach the detection target through the first opening portion 36. In addition, by using the light shielding member in the first plate body 32A, stray light that affects a detection accuracy can be reduced.

The vent hole 30 connects a space of the vent hole 30 to the buffer space 28, and opens on a surface of the first plate body 32A in the Z1 direction (an outer surface of the test strip 12). The vent hole 30 is a hole for discharging air in the flow path 26 and the buffer space 28 to the outside of the main body portion 20 when the blood is guided from the intake portion 24 to the flow path 26. The vent hole 30 is provided independently at a position away from the first opening portion 36 by a predetermined distance in the arrow X2 direction. The vent hole 30 is located substantially at the center of the first plate body 32A in the width direction (the arrow Y direction).

The second plate body 32B is a film-shaped member stacked on the other side (the arrow Z2 direction) of the test strip 12 in the thickness direction with respect to the first plate body 32A. The second plate body 32B is provided with a second cutout portion 24b and a first buffer hole 28a.

The second cutout portion 24b forms a part of the intake portion 24. The second cutout portion 24b is formed at an end portion of the second plate body 32B in the arrow X1 direction. The second cutout portion 24b is formed in a rectangular shape in the plan view from the arrow Z direction. The second cutout portion 24b communicates with the first cutout portion 24a in the arrow Z2 direction. The second cutout portion 24b is formed in the same size and shape as the first cutout portion 24a.

The first buffer hole 28a forms a part of the buffer space 28. The first buffer hole 28a is a rectangular through-hole penetrating the second plate body 32B in the thickness direction. Specifically, the first buffer hole 28a is formed in a square shape in the plan view from the arrow Z direction. The first buffer hole 28a may have a rectangular shape extending in the arrow X direction, or may have a rectangular shape extending in the arrow Y direction.

The first buffer hole 28a is provided independently at a position away from the second cutout portion 24b by a predetermined distance in the arrow X2 direction. The first buffer hole 28a is located substantially at a center of the second plate body 32B in the width direction. Wall portions of the second plate body 32B exist on both sides of the first buffer hole 28a in the arrow Y direction. The first buffer hole 28a is formed at a position facing the vent hole 30. That is, the first buffer hole 28a communicates with the vent hole 30 in the arrow Z2 direction. The first buffer hole 28a is formed to have a size larger than that of the vent hole 30 in the plan view from the arrow Z direction.

A wall portion 38 of the second plate body 32B between the second cutout portion 24b and the first buffer hole 28a covers a side of the first opening portion 36 in the Z2 direction (see FIG. 4). The wall portion 38 is formed to be transparent so as to allow light transmitting through the first opening portion 36 to pass therethrough. The entire second plate body 32B is formed to be transparent (colorless transparent or colored transparent). In the second plate body 32B, only the wall portion 38 may be formed to be transparent, and a part other than the wall portion 38 may be formed to be opaque.

The third plate body 32C is a film-shaped member stacked in the arrow Z2 direction with respect to the second plate body 32B. The third plate body 32C is provided with a third cutout portion 24c, a first flow path groove 26a, and a second buffer hole 28b.

The third cutout portion 24c forms a part of the intake portion 24. The third cutout portion 24c is formed at an end portion of the third plate body 32C in the arrow X1 direction. The third cutout portion 24c is formed in a rectangular shape in the plan view from the arrow Z direction. The third cutout portion 24c communicates with the second cutout portion 24b in the arrow Z2 direction. The third cutout portion 24c is formed in the same size and shape as each of the first cutout portion 24a and the second cutout portion 24b.

The first flow path groove 26a forms a part of the flow path 26. The first flow path groove 26a linearly extends along the third plate body 32C in a longitudinal direction. The first flow path groove 26a penetrates the third plate body 32C in the thickness direction. The first flow path groove 26a is located substantially at a center of the third plate body 32C in the width direction. One end (an end in the arrow X1 direction, a starting end) of the first flow path groove 26a communicates with the third cutout portion 24c. The other end (an end in the arrow X2 direction, a terminal end) of the first flow path groove 26a communicates with the second buffer hole 28b. That is, the third cutout portion 24c, the first flow path groove 26a, and the second buffer hole 28b form one continuous space.

The first flow path groove 26a is formed to be narrower than the third cutout portion 24c. The first flow path groove 26a is covered in the arrow Z1 direction by the wall portion 38 of the second plate body 32B (see FIG. 4). That is, the wall portion 38 of the second plate body 32B blocks a space between the first flow path groove 26a and the first opening portion 36 in a liquid-tight manner. The wall portion 38 of the second plate body 32B serves as a top surface of the flow path 26 in the arrow Z1 direction.

The second buffer hole 28b forms a part of the buffer space 28. The second buffer hole 28b is a rectangular through-hole penetrating the third plate body 32C in the thickness direction. Specifically, the second buffer hole 28b is formed in a square shape in the plan view from the arrow Z direction. The second buffer hole 28b may have a rectangular shape extending in the arrow X direction, or may have a rectangular shape extending in the arrow Y direction.

The second buffer hole 28b is located substantially at a center of the third plate body 32C in the width direction. Wall portions of the third plate body 32C exist on both sides of the second buffer hole 28b in the arrow Y direction. The second buffer hole 28b is formed at a position facing the first buffer hole 28a. That is, the second buffer hole 28b communicates with the first buffer hole 28a in the arrow Z2 direction. The second buffer hole 28b is formed in the same size and shape as the first buffer hole 28a. The second buffer hole 28b is formed to be wider than the first flow path groove 26a. In other words, a width of the second buffer hole 28b is widened to both sides in the arrow Y direction with respect to the first flow path groove 26a in the plan view from the arrow Z direction.

The fourth plate body 32D is a film-shaped member stacked in the arrow Z2 direction with respect to the third plate body 32C. The fourth plate body 32D is provided with a fourth cutout portion 24d, a second flow path groove 26b, a reagent disposing hole 40, and a third buffer hole 28c.

The fourth cutout portion 24d forms a part of the intake portion 24. The fourth cutout portion 24d is formed at an end portion of the fourth plate body 32D in the arrow X1 direction. The fourth cutout portion 24d is formed in a rectangular shape in the plan view from the arrow Z direction. The fourth cutout portion 24d communicates with the third cutout portion 24c in the arrow Z2 direction. The fourth cutout portion 24d is formed in the same shape and size as each of the first cutout portion 24a, the second cutout portion 24b, and the third cutout portion 24c.

The second flow path groove 26b forms a part of the flow path 26. The second flow path groove 26b linearly extends along the fourth plate body 32D in the longitudinal direction. The second flow path groove 26b penetrates the fourth plate body 32D in the thickness direction. The second flow path groove 26b is located substantially at a center of the fourth plate body 32D in the width direction. One end (an end in the arrow X1 direction, a starting end) of the second flow path groove 26b communicates with the fourth cutout portion 24d. The second flow path groove 26b is terminated at a position of the reagent disposing hole 40.

The second flow path groove 26b is formed to be narrower than the fourth cutout portion 24d. The second flow path groove 26b is formed at a position facing the first flow path groove 26a. That is, the second flow path groove 26b communicates with the first flow path groove 26a in the arrow Z2 direction. A width of the second flow path groove 26b along the arrow Y direction is the same as a width of the first flow path groove 26a along the arrow Y direction. In the arrow X direction, a total length of the second flow path groove 26b. is shorter than a total length of the first flow path groove 26a (see FIG. 4).

The reagent disposing hole 40 is a space in which the reagent piece 22 can be disposed, and the reagent disposing hole 40 is provided between the second flow path groove 26b and the third buffer hole 28c. The reagent disposing hole 40 penetrates the fourth plate body 32D in the thickness direction and extends in a rectangular shape over a total width (a total length in the arrow Y direction) of the fourth plate body 32D. The reagent disposing hole 40 faces an end portion of the first flow path groove 26a in the arrow X2 direction.

The fourth plate body 32D is divided into a first member 42a, a second member 42b, and a third member 42c by the fourth cutout portion 24d, the second flow path groove 26b, and the reagent disposing hole 40. The first member 42a and the second member 42b are disposed on both sides of the second flow path groove 26b in the arrow Y direction. Side surfaces of the first member 42a and the second member 42b on central axis sides form a part of a wall of the flow path 26. The third member 42c is disposed in the arrow X2 direction of the first member 42a and the second member 42b so as to sandwich the reagent disposing hole 40.

The third buffer hole 28c forms a part of the buffer space 28. The third buffer hole 28c is a rectangular through-hole penetrating the fourth plate body 32D in the thickness direction. Specifically, the third buffer hole 28c is formed in a square shape in the plan view from the arrow Z direction. The third buffer hole 28c may have a rectangular shape extending in the arrow X direction, or may have a rectangular shape extending in the arrow Y direction.

The third buffer hole 28c is located substantially at a center of the fourth plate body 32D in the width direction. Wall portions of the fourth plate body 32D exist on both sides of the third buffer hole 28c in the arrow Y direction. The third buffer hole 28c is formed at a position facing the second buffer hole 28b. That is, the third buffer hole 28c communicates with the second buffer hole 28b in the arrow Z2 direction. The third buffer hole 28c is formed in the same size and shape as each of the first buffer hole 28a and the second buffer hole 28b.

The fifth plate body 32E is a film-shaped member stacked in the arrow Z2 direction with respect to the fourth plate body 32D. The fifth plate body 32E is provided with a reagent insertion hole 44 and a fourth buffer hole 28d.

The reagent insertion hole 44 is formed to face the reagent disposing hole 40 and to have the same shape as the reagent disposing hole 40. The reagent insertion hole 44 faces the reagent disposing hole 40 in the arrow Z2 direction. That is, the reagent insertion hole 44 penetrates the fifth plate body 32E in the thickness direction and extends over a total width (a total length in the arrow Y direction) of the fifth plate body 32E.

The fifth plate body 32E is divided into a first member 46a and a second member 46b by the reagent insertion hole 44. The first member 46a is disposed in the arrow X1 direction so as to sandwich the reagent insertion hole 44 with the second member 46b. The first member 46a covers the fourth cutout portion 24d and the second flow path groove 26b from the arrow Z2 direction in a liquid-tight manner (see FIG. 4). An end portion of the first member 46a in the arrow X1 direction is formed in a semicircular shape.

The fourth buffer hole 28d forms a part of the buffer space 28. The fourth buffer hole 28d is a rectangular through-hole penetrating the fifth plate body 32E in the thickness direction. Specifically, the fourth buffer hole 28d is formed in a square shape in the plan view from the arrow Z direction. The fourth buffer hole 28d may have a rectangular shape extending in the arrow X direction, or may have a rectangular shape extending in the arrow Y direction.

The fourth buffer hole 28d is located substantially at a center of the fifth plate body 32E in the width direction. Wall portions of the fifth plate body 32E exist on both sides of the fourth buffer hole 28d in the arrow Y direction. The fourth buffer hole 28d is formed at a position facing the third buffer hole 28c. That is, the fourth buffer hole 28d communicates with the third buffer hole 28c in the arrow Z2 direction. The fourth buffer hole 28d is formed in the same size and shape as each of the first buffer hole 28a, the second buffer hole 28b, and the third buffer hole 28c.

The sixth plate body 32F is a film-shaped member stacked in the arrow Z2 direction with respect to the fifth plate body 32E. The sixth plate body 32F is a planar member disposed at an end in the thickness direction (an end in the arrow Z2 direction) of the test strip 12. The sixth plate body 32F forms one surface of the test strip 12. The sixth plate body 32F covers the fourth buffer hole 28d from the arrow Z2 direction in a liquid-tight manner (see FIG. 4) . The sixth plate body 32F is provided with a second opening portion 48.

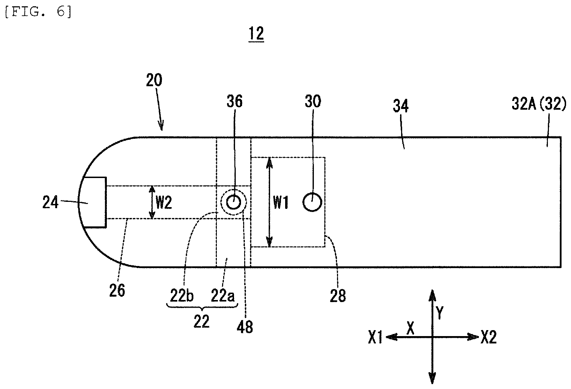

The second opening portion 48 is a circular through-hole through which the measurement light is transmitted in the thickness direction of the test strip 12. The second opening portion 48 is located in the arrow Z2 direction of the first opening portion 36. A diameter of the second opening portion 48 is larger than a diameter of the first opening portion 36 (see FIG. 6). In other words, the second opening portion 48 is provided such that the entire first opening portion 36 is located inside the second opening portion 48 when viewed from the arrow Z direction. The sixth plate body 32F may be provided with the transparent portion (the light guiding portion) capable of transmitting the measurement light in place of the second opening portion 48.

In the main body portion 20 configured as described above, the intake portion 24 is formed by the first cutout portion 24a, the second cutout portion 24b, the third cutout portion 24c, and the fourth cutout portion 24d. The flow path 26 is formed by the first flow path groove 26a and the second flow path groove 26b. The length of the intake portion 24 in the width direction is larger than the length of the flow path 26 in the width direction. The buffer space 28 is formed by the first buffer hole 28a, the second buffer hole 28b, the third buffer hole 28c, and the fourth buffer hole 28d. A surface on one end (the arrow Z2 direction) of the buffer space 28 is sealed by the sixth plate body 32F, and a surface on the other end (the arrow Z1 direction) of the buffer space 28 is covered by the first plate body 32A including the vent hole 30. A length of the buffer space 28 in the width direction is larger than either one of the length of the flow path 26 in the width direction and the length of the intake portion 24 in the width direction.

In FIG. 4, a starting end of the flow path 26 communicates with the intake portion 24. A terminal end of the flow path 26 (the terminal end of the first flow path groove 26a) communicates with the buffer space 28. A reagent for analyte detection is disposed at any position between the starting end and the terminal end of the flow path 26. That is, the buffer space 28 is present downstream of the reagent in the flow path 26. A space of the flow path 26 is connected to the buffer space 28 in a direction substantially perpendicular to the buffer space 28. The buffer space 28 is formed in a rectangular parallelepiped shape (hexahedral shape). The buffer space 28 can store blood when the blood leaks from the terminal end of the flow path 26 (the first flow path groove 26a). A volume of the buffer space 28 is larger than a volume of the flow path 26. Accordingly, the blood can be inhibitedfrom leaking from the vent hole 30 by the buffer space 28.

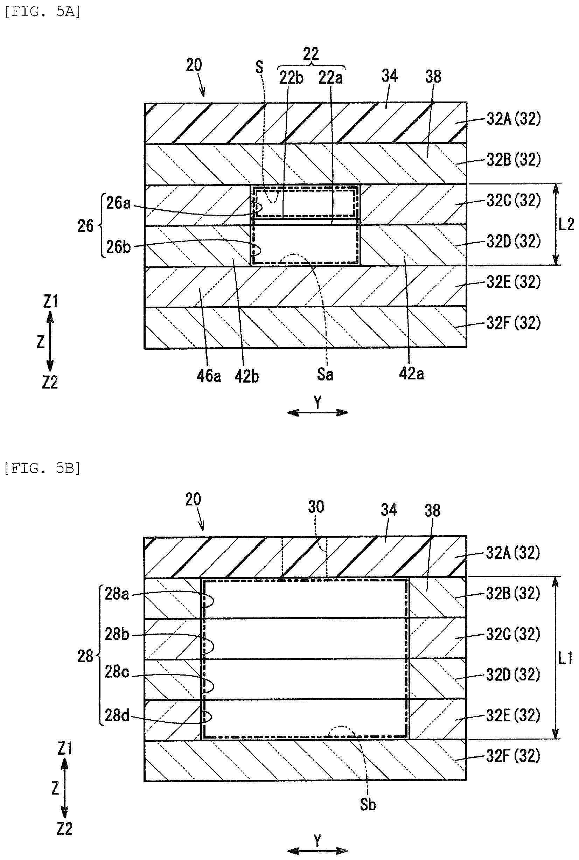

As illustrated in FIG. 4 and FIGS. 5A and 5B, a cross-sectional area of the buffer space 28 is larger than a cross-sectional area S of the flow path 26 in a connection portion between the buffer space 28 and the flow path 26. In a cross section along the arrow X direction passing through a center of the flow path 26 along the width direction, a total thickness (the length along the arrow Z direction) of the buffer space 28 is preferably 3 times to 10 times a thickness (the length along the arrow Z direction) of the flow path 26 in a region where the flow path 26 and the buffer space 28 are connected. An increment in the thickness of the buffer space 28 with respect to a thickness of a terminal cross section of the flow path 26 is preferably 1.1 times to 10 times, more preferably 3.3 times to 5 times the thickness of the terminal cross section of the flow path 26 in each of the Z1 direction and the Z2 direction. In other words, in the region where the flow path 26 and the buffer space 28 are connected (a boundary region between the flow path 26 and the buffer space 28), the buffer space 28 preferably extends in the arrow Z1 direction with respect to the flow path 26 by a length of 1 time to 10 times the length of the flow path 26 in the arrow Z direction, and more preferably extends by a length of 3.3 times to 5 times the length of the flow path 26 in the arrow Z direction. In the region where the flow path 26 and the buffer space 28 are spatially connected, the buffer space 28 preferably extends in the arrow Z2 direction with respect to the flow path 26 by a length of 1 time to 10 times the length of the flow path 26 in the arrow Z direction, and more preferably extends by a length of 3.3 times to 5 times the length of the flow path 26 in the arrow Z direction.

A total length of the buffer space 28 in the width direction (the arrow Y direction) is preferably 2 times to 5 times the length of the terminal end of the flow path 26 in the width direction (the arrow Y direction). An increment in a width of the buffer space 28 with respect to the width direction of the terminal end of the flow path 26 is preferably 0.5 times to 2 times the width direction of the terminal end of the flow path 26 on either side in the Y direction. In other words, in the region where the flow path 26 and the buffer space 28 are spatially connected, the buffer space 28 preferably extends to one side in the arrow Y direction with respect to the flow path 26 by a length of 0.5 times to 2 times the length of the flow path 26 in the width direction. In the region where the flow path 26 and the buffer space 28 are spatially connected, the buffer space 28 preferably extends to the other side in the arrow Y direction with respect to the flow path 26 by a length of 0.5 times to 2 times the length of the flow path 26 in the width direction.

In the boundary region where the buffer space 28 and the flow path 26 are spatially connected, when cross sections in a direction orthogonal to the arrow X direction are compared, a cross-sectional area Sb of the buffer space 28 is larger than the cross-sectional area S of the flow path 26. The cross-sectional area Sb of the buffer space 28 is larger than a maximum flow path cross-sectional area Sa of the flow path 26. Here, the maximum flow path cross-sectional area Sa of the flow path 26 is a sum of a flow path cross-sectional area of the first flow path groove 26a and a flow path cross-sectional area of the second flow path groove 26b.

In other words, the buffer space 28 is connected to the flow path 26 to form a space expanding on both sides in the arrow Y direction and both sides in the arrow Z direction, in a portion where the buffer space 28 is spatially connected to the flow path 26. That is, as illustrated in FIG. 6, a width W1 of the buffer space 28 along the arrow Y direction is larger than a width W2 of the flow path 26 along the arrow Y direction. As illustrated in FIGS. 5A and 5B, a length L1 of the buffer space 28 in the arrow Z direction is larger than a maximum length L2 of the flow path 26 in the arrow Z direction. The volume of the buffer space 28 is larger than the volume of the flow path 26. In FIG. 6, the vent hole 30 is located at an end of the buffer space 28 in the arrow X2 direction and is located substantially at a center of the buffer space 28 in the arrow Y direction.

In FIG. 4, a surface of the second plate body 32B in the arrow Z2 direction and a surface of the fifth plate body 32E in the arrow Z1 direction are subjected to a hydrophilization treatment (not illustrated). Accordingly, the blood can easily flow in the flow path 26 sandwiched between the second plate body 32B and the fifth plate body 32E.

As illustrated in FIGS. 3 and 4, the reagent piece 22 includes a support base 22a and a reagent portion 22b provided on the support base 22a. The support base 22a is formed in a rectangular shape in the plan view from the arrow Z direction. The reagent portion 22b is located substantially at a central portion of the support base 22a in a longitudinal direction. Both sides of the reagent portion 22b in the longitudinal direction of the support base 22a are attached to a surface of the third plate body 32C in the arrow Z2 direction such that the reagent portion 22b is located inside the first flow path groove 26a.

The support base 22a is formed in a rectangular shape in which a long side extends in a lateral direction (the width direction, that is, the arrow Y direction) of the test strip 12, while a short side extends in the longitudinal direction (the arrow X direction). As in the case of the plate body 32, a transparent film material can be used for the support base 22a. By adjusting a thickness of the support base 22a, the thickness of the flow path 26 in the arrow Z direction may be changed in the middle of the flow path 26. In the present embodiment, in a part where the reagent portion 22b is disposed in the flow path 26, the thickness of the flow path 26 in the arrow Z direction is reduced. Therefore, an area of a cross section of the flow path 26, which is orthogonal to the arrow X direction, is smallest on the support base 22a.

The reagent disposing hole 40 extends over the total width of the fourth plate body 32D, and the reagent insertion hole 44 extends over the total width of the fifth plate body 32E. Therefore, in the present embodiment, a width of the test strip 12 in the arrow Y direction can be narrowed without narrowing the widths of the reagent disposing hole 40 and the reagent insertion hole 44 in the arrow Y direction. That is, the test strip 12 can be made compact while sufficiently ensuring an adhesion area of the support base 22a to the third plate body 32C. More specifically, a length of the support base 22a along the arrow Y direction is larger than the width of the flow path 26 along the arrow Y direction, and is the same as the length of the test strip 12 in the lateral direction or less than the length of the test strip 12 in the lateral direction. Accordingly, even if the length of the test strip 12 in the arrow Y direction is reduced to 10 mm or less, the support base 22a can reliably adhere to the third plate body 32C.

The reagent portion 22b supports a reagent that reacts with the sample in at least a part of the flow path 26. The reagent portion 22b is applied to the support base 22a without blocking the inside of the flow path 26. Various polymers and carriers may be further disposed on the support base 22a depending on properties of the reagent and a measurement system. The reagent portion 22b overlaps the first opening portion 36 when viewed from the arrow Z direction in a state where the support base 22a is disposed in the reagent disposing hole 40 (see FIG. 6). Therefore, the measurement light of the blood glucose meter 16 is emitted toward the reagent portion 22b. When the measurement light (transmitted light) transmitted through the detection target is detected, it is preferable not to use the carrier such as a porous member. In this case, a reagent solution is directly applied to the support base 22a by using a known means such as ink jet and dried to form the reagent portion 22b.

The reagent piece 22 is configured as a member separate from the plate body 32, but the invention is not limited thereto. For example, the reagent portion 22b may be configured by applying the reagent at an appropriate position on a predetermined plate body 32 (for example, the surface of the second plate body 32B in the arrow Z2 direction). The reagent may be applied to any portion in the second flow path groove 26b or in a region between the second flow path groove 26b and the third buffer hole 28c. Alternatively, the reagent may be applied onto any one of wall surfaces constituting the region where the flow path 26 and the reagent disposing hole 40 face each other to form the reagent portion 22b. In this case, the reagent portion 22b is provided on a part of a surface of the second plate body 32B in the Z2 direction, which faces the reagent disposing hole 40. Even when the reagent piece 22 is not used, a thickness of a space above a portion to which the reagent is applied is preferably configured to be smaller than the thickness of the flow path 26. Accordingly, the blood can quickly flow into a space above the reagent portion 22b from the flow path 26.

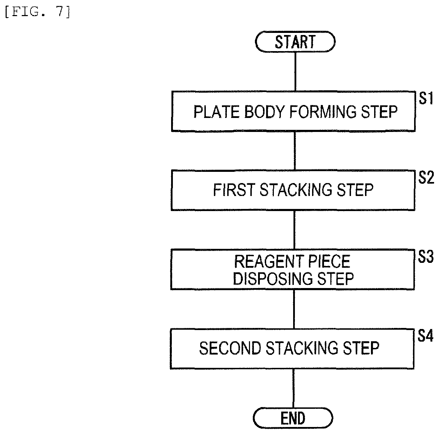

As illustrated in FIG. 7, a method of manufacturing the test strip 12 described above includes a plate body forming step, a first stacking step, a reagent piece disposing step, and a second stacking step. In the plate body forming step (step S1), a film member is subjected to a treatment (punching, or the like) to form the first plate body 32A, the second plate body 32B, the third plate body 32C, the fourth plate body 32D, the fifth plate body 32E, and the sixth plate body 32F. In the present embodiment, at least six plate bodies 32 are stacked to form the test strip 12.

In the first stacking step (step S2), the first plate body 32A, the second plate body 32B, the third plate body 32C, the fourth plate body 32D, and the fifth plate body 32E are bonded together while being stacked by using a double-sided tape or an adhesive. In the reagent piece disposing step (step S3), the reagent piece 22 is inserted into the reagent disposing hole 40 from the reagent insertion hole 44 of the fifth plate body 32E. At this time, both sides of the reagent portion 22b in the support base 22a are pasted to a surface of the third plate body 32C in the arrow Z2 direction. Accordingly, the reagent piece 22 is fixed to the third plate body 32C. In the second stacking step (step S4), the sixth plate body 32F is bonded to the fifth plate body 32E. Accordingly, the test strip 12 is manufactured.

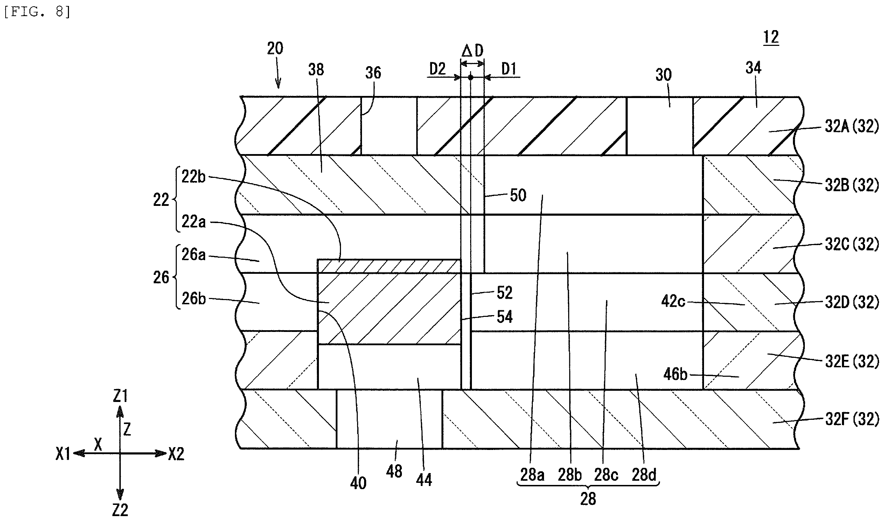

In the test strip 12 manufactured in this manner, as illustrated in FIG. 8, in the first stacking step, the plate bodies 32 may be displaced in the arrow X direction when the plate bodies 32 are bonded to each other. That is, an end (a first buffer end 50) of the first buffer hole 28a of the second plate body 32B in the arrow X1 direction and an end (a second buffer end 52) of the third buffer hole 28c of the fourth plate body 32D in the arrow X1 direction may be displaced by a first distance D1 in the arrow X direction.

In the reagent piece disposing step, when the support base 22a is pasted to the surface of the third plate body 32C in the arrow Z2 direction, the reagent piece 22 may be displaced in the arrow X direction with respect to the fourth plate body 32D. That is, an end surface 54 of the reagent piece 22 in the arrow X2 direction and the second buffer end 52 may be displaced by a second distance D2 in the arrow X direction.

In this case, the first buffer end 50 is displaced by a predetermined displacement amount ΔD (ΔD = D1 + D2) with respect to the end surface 54 of the reagent piece 22. The displacement amount ΔD is a displacement in the arrow X direction generated at a connection end of the flow path 26 in the region where the buffer space 28 and the flow path 26 are spatially connected. More specifically, the displacement amount corresponds to a displacement length in the arrow X direction, which is generated between upper and lower surfaces of the flow path 26 at the terminal end of the flow path 26. In the present embodiment, the displacement amount ΔD is preferably set to less than 0.24 mm, more preferably 0.22 mm or less, and still more preferably 0.10 mm or less.

Next, the blood glucose meter 16 to which the test strip 12 is attached will be described. As illustrated in FIG. 1, the blood glucose meter 16 is configured in a reuse type capable of performing blood glucose measurement repeatedly. A housing 60 of the blood glucose meter 16 includes a box body portion 64 that has a size easy for the user to grip and operate, and accommodates a control unit 62 of the blood glucose meter 16 therein, and a cylindrical light measurement unit 66 that protrudes from the box body portion 64 and accommodates the measurement unit 18 of an optical system therein.

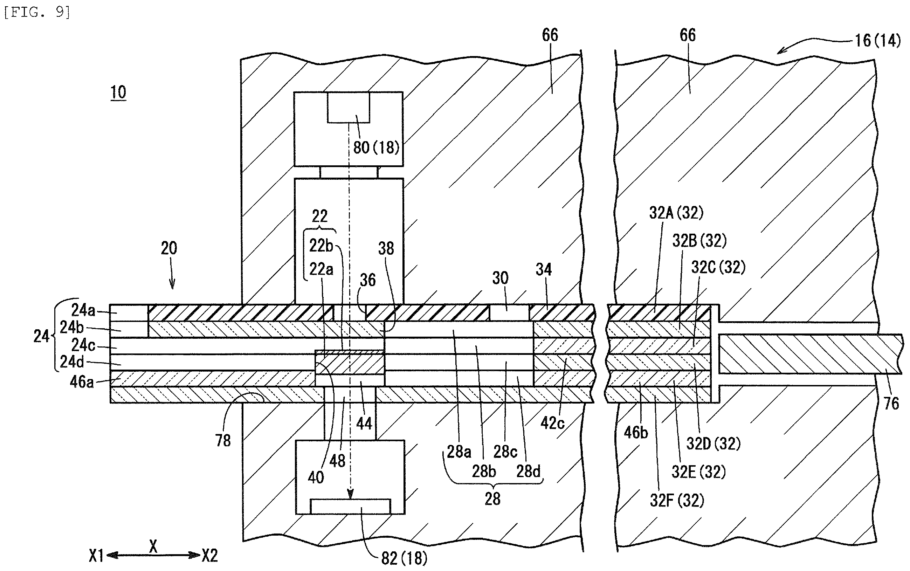

A power supply button 68, an operation button 70, and a display 72 are provided on an upper surface of the box body portion 64, and an eject lever 74 as an operation unit for removing the test strip 12 after use is provided on an upper surface of the light measurement unit 66. The eject lever 74 is provided so as to be movable along an extending direction of the light measurement unit 66, and is connected to an eject pin 76 (see FIG. 9) provided in the light measurement unit 66.

As illustrated in FIG. 9, the light measurement unit 66 is provided with an insertion hole 78 into which the test strip 12 is inserted. The measurement unit 18 optically detects glucose in the blood. The measurement unit 18 includes a light emitting unit 80 and a light receiving unit 82. The light emitting unit 80 and the light receiving unit 82 are disposed so as to face each other with the insertion hole 78 sandwiched therebetween.

As the light emitting unit 80, an LED, an organic EL, a laser diode, or the like is used. In a state where the test strip 12 is attached to the insertion hole 78, the light emitting unit 80 emits light with a predetermined wavelength toward the first opening portion 36 of the test strip 12. As the light receiving unit 82, for example, a photodiode is used. The light receiving unit 82 receives the light transmitted through the test strip 12 (the reagent portion 22b).

The control unit 62 of the blood glucose meter 16 is configured with a control circuit (a computer) (not illustrated) including a converter, a processor, a memory, and an input/output interface. For example, under the operation of the user, the control unit 62 drives the measurement unit 18 to calculate the blood glucose level based on a signal corresponding to an amount of glucose (or concentration) in the blood. The calculated blood glucose level is displayed on the display 72.

Next, the measurement of the blood glucose level using the test strip 12 according to the present embodiment will be described.

As illustrated in FIG. 9, the user inserts the test strip 12 into the insertion hole 78 of the blood glucose meter 16 and locates the reagent portion 22b of the test strip 12 between the light emitting unit 80 and the light receiving unit 82. Next, the user attaches a small amount of blood to the intake portion 24 of the test strip 12. The blood flows in the flow path 26 toward the detection target position by the capillary force.

Next, the user operates the operation button 70 of the blood glucose meter 16 to start measuring the blood glucose level. Then, the measurement light emitted from the light emitting unit 80 transmits through the first opening portion 36, the wall portion 38 of the second plate body 32B, the first flow path groove 26a, the reagent portion 22b, the support base 22a, the reagent insertion hole 44, and the second opening portion 48, and is received by the light receiving unit 82. The control unit 62 of the blood glucose meter 16 calculates the blood glucose level based on an output signal from the light receiving unit 82 and displays the blood glucose level on the display 72. Accordingly, the measurement of the blood glucose level ends.

As the blood flows through the flow path 26, the air in the flow path 26 and the buffer space 28 is vented to the outside of the main body portion 20 through the vent hole 30. The blood that has reached the reagent piece 22 flows into a space above the reagent portion 22b. When the blood reaches the reagent portion 22b, the reagent portion 22b rapidly dissolves in the blood, and a reaction between glucose and the reagent proceeds. In the connection portion between the buffer space 28 and the flow path 26, the cross-sectional area Sb of the buffer space 28 is larger than the maximum flow path cross-sectional area Sa of the flow path 26. In other words, in the region where the buffer space 28 and the flow path 26 are spatially connected, the cross-sectional area Sb of the buffer space 28 is larger than the cross-sectional area S of the flow path 26. Therefore, the blood in the flow path 26 is difficult to be drawn into the buffer space 28 due to a capillary phenomenon. Therefore, the blood is prevented from flowing from the flow path 26 into the buffer space 28. This is because, when the sample reaches the terminal end of the flow path 26, the blood is inhibited from leaking into the buffer space 28 beyond the terminal end of the flow path 26 due to an action of surface tension of the blood, which is applied to the terminal cross section of the flow path 26 from a buffer space 28 side. As described above, by providing the buffer space 28, it is possible to omit an arrangement of a volume swelling member, an embolization member, and a micro flow path for inhibitingthe sample from leaking from the test strip 12, and a special surface treatment such as a water repellent treatment.

The volume of the buffer space 28 is 2 times to 5 times the volume of the flow path 26. Even if the blood in the flow path 26 flows into the buffer space 28, the blood can be sufficiently stored in the buffer space 28. Therefore, the blood is inhibitedfrom leaking from the vent hole 30. Accordingly, the inside of the blood glucose meter 16 is inhibitedfrom being contaminated with blood. An area of the vent hole 30 is 10 times to 30 times the maximum flow path cross-sectional area Sa. By making the area of the vent hole 30 sufficiently larger than the maximum flow path cross-sectional area Sa, the air can be quickly vented from the vent hole 30 when the blood flows into the flow path 26.

The test strip 12 according to the present embodiment achieves the following effects.

The main body portion 20 is provided with the buffer space 28 communicating with the terminal end of the flow path 26, and the vent hole 30 opened at an outer surface of the main body portion 20 and communicating with the buffer space 28. In the region where the buffer space 28 and the flow path 26 are spatially connected, the cross-sectional area Sb of the buffer space 28 is larger than the cross-sectional area S of the flow path 26.

According to such a configuration, in the region where the buffer space 28 and the flow path 26 are spatially connected, the cross-sectional area Sb of the buffer space 28 is larger than the cross-sectional area S of the flow path 26. Therefore, at the connection portion between the buffer space 28 and the flow path 26, which is the terminal end of the flow path 26, an interfacial tension component in a flow direction is reduced for blood in the flow path 26. Accordingly, the capillary force that causes the blood to be drawn into the buffer space 28 is less likely to occur. Even if the blood in the flow path 26 flows into the buffer space 28, the blood can be kept in the buffer space 28. Accordingly, the blood can be inhibited from leaking from the vent hole 30 to the outside of the main body portion 20 while avoiding an increase in costs.

The buffer space 28 is formed in a rectangular parallelepiped shape.

According to such a configuration, the blood can be effectively inhibited from leaking from the vent hole 30 to the outside of the main body portion 20.

The flow path 26 is connected to the buffer space 28 in a direction substantially perpendicular to the buffer space 28.

According to such a configuration, in the connection portion between the buffer space 28 and the flow path 26, the surface tension of the blood that has reached the cross section of the flow path 26 can act uniformly on the cross section of the flow path 26. Accordingly, the blood can be effectively inhibited from flowing from the flow path 26 into the buffer space 28.

The main body portion 20 is formed by stacking a plurality of film members. In an arrangement direction of the flow path 26 and the buffer space 28, the displacement amount ΔD between an end (the end surface 54) of the reagent portion 22b on the buffer space 28 side and an end (the first buffer end 50) of the buffer space 28 on a flow path 26 side is 0.22 mm or less.

According to such a configuration, the test strip 12 can be manufactured by the plurality of plate bodies 32 while more effectively inhibiting the blood from flowing from the flow path 26 into the buffer space 28.

In the arrangement direction of the flow path 26 and the buffer space 28, a maximum displacement amount between a terminal end of an upper surface of the flow path 26 and a terminal end of a lower surface of the flow path 26 is less than 0.24 mm.

According to such a configuration, in the connection portion between the buffer space 28 and the flow path 26, the surface tension of the blood that has reached the terminal end of the flow path 26 can act uniformly in the cross section of the flow path 26. Furthermore, interfacial tension of the blood that has reached the terminal end of the flow path 26 can act uniformly on the cross section of the flow path 26.

In the arrangement direction of the flow path 26 and the buffer space 28, a maximum displacement amount between the end of the reagent portion 22b on the buffer space 28 side and the end of the buffer space 28 on the flow path 26 side is less than 0.24 mm.

According to such a configuration, by reducing a positional displacement between the first buffer end 50 and the end surface 54, a decrease in an action of the interfacial tension acting on an interface between the blood and the flow path cross section can be inhibited. Accordingly, the blood can be effectively inhibited from flowing from the flow path 26 into the buffer space 28.

Next, a first test performed to check the effects of the invention will be described.

### [Sample]

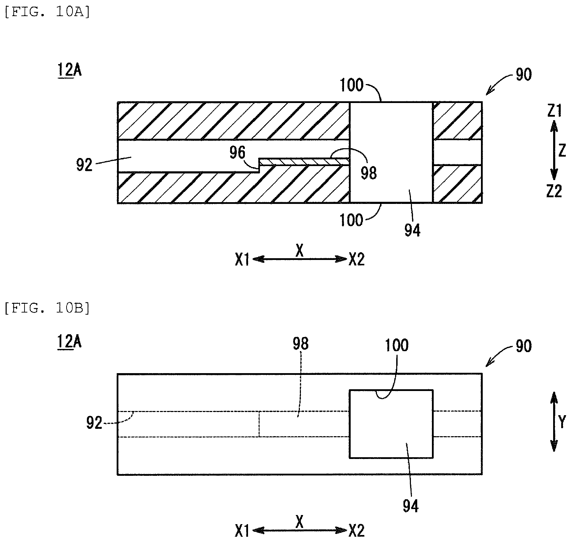

One test strip 12A (Example 1) according to the invention was prepared, and four test strips 102A to 102D (Comparative Examples 1 to 4) according to Comparative Examples were prepared. The test strip has a width of 6 mm, a length of 24 mm, and a thickness of about 550 µm. A volume in a flow path is about 0.9 µL. As illustrated in FIGS. 10A and 10B, a flow path 92 and a buffer space 94 communicating with the flow path 92 are formed in a main body portion 90 of the test strip 12A according to Example 1. A starting end of the flow path 92 is opened at an end surface of the main body portion 90 in an arrow X1 direction. A terminal end of the flow path 92 communicates with the buffer space 94. A step portion 96 is formed on an inner surface of the flow path 92. A thickness of the flow path 92 in an arrow Z direction is reduced due to the step portion 96. A reagent portion 98 is applied onto the step portion 96. The reagent portion 98 is the same as the reagent portion 22b described above.

In Example 1, the buffer space 94 penetrates the main body portion 90 in a thickness direction. The buffer space 94 is formed in a rectangular shape when viewed from the thickness direction of the main body portion 90. In other words, the buffer space 94 has a rectangular parallelepiped shape. A width of the buffer space 94 along an arrow Y direction is larger than a width of the flow path 92 along the arrow Y direction (see FIG. 10B). A length of the buffer space 94 in the arrow Z direction is larger than a length of the flow path 92 in the arrow Z direction (see FIG. 10A). In other words, in a connection portion between the buffer space 94 and the flow path 92, a cross-sectional area of the buffer space 94 is larger than a maximum flow path cross-sectional area of the flow path 92. Both opening portions of the buffer space 94, which are opened at outer surfaces of the main body portion 90, function as vent holes 100 through which air is discharged.

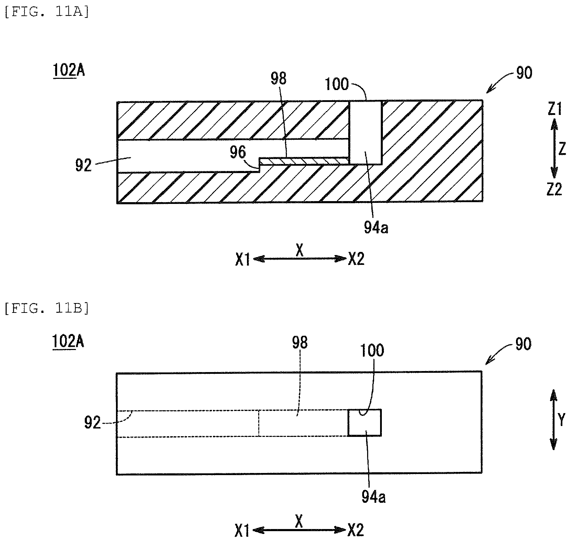

In Comparative Examples 1 to 4, a shape and a size of the buffer space 94 of the test strip 12A according to Example 1 were changed. As illustrated in FIGS. 11A and 11B, in the test strip 102A according to Comparative Example 1, a buffer space 94a is enlarged only on an arrow Z1 direction side in a connection portion between the flow path 92 and the buffer space 94a. The buffer space 94a is opened only on a front surface of the main body portion 90 on the arrow Z1 direction side. An opening portion of the buffer space 94a functions as the vent hole 100. The buffer space 94a is formed in a rectangular shape when viewed from a thickness direction (the arrow Z1 direction) of the main body portion 90. A width of the buffer space 94a (a length in the arrow Y direction) is the same as a width of the flow path 92 (see FIG. 11B). The buffer space 94 has a rectangular parallelepiped shape.

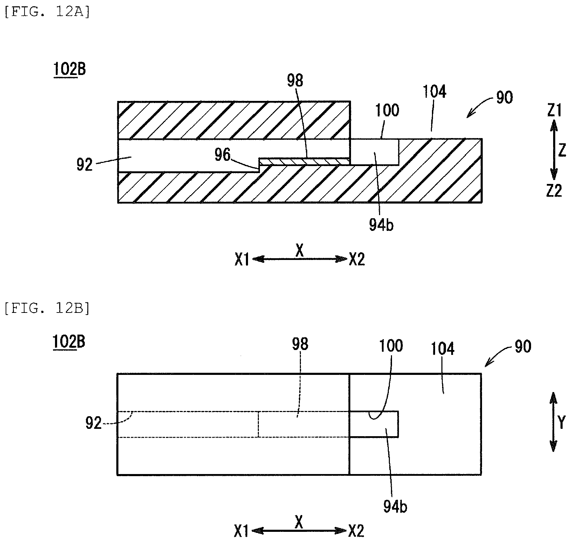

As illustrated in FIGS. 12A and 12B, in the test strip 102B according to Comparative Example 2, a buffer space 94b is formed in a rectangular shape when viewed from a thickness direction (the arrow Z1 direction) of the main body portion 90. The buffer space 94b is enlarged only in the arrow Z1 direction in a connection portion between the flow path 92 and the buffer space 94b. An end of the buffer space 94 in the arrow X1 direction and an end of the buffer space 94b in the arrow X2 direction differ in a thickness in the arrow Z direction by a thickness of a plate body forming a surface of the main body portion 90 in the arrow Z1 direction. A width of the buffer space 94b is the same as the width of the flow path 92 (see FIG. 12B). The main body portion 90 is provided with an open space 104 communicating with the buffer space 94b. The open space 104 is formed by cutting out a part of a portion of the main body portion 90 in the arrow X2 direction relative to the flow path 92. The open space 104 is located on a side opposite to the flow path 92 with the reagent portion 98 sandwiched therebetween. A connection portion of the buffer space 94b that is connected to the open space 104 functions as the vent hole 100.

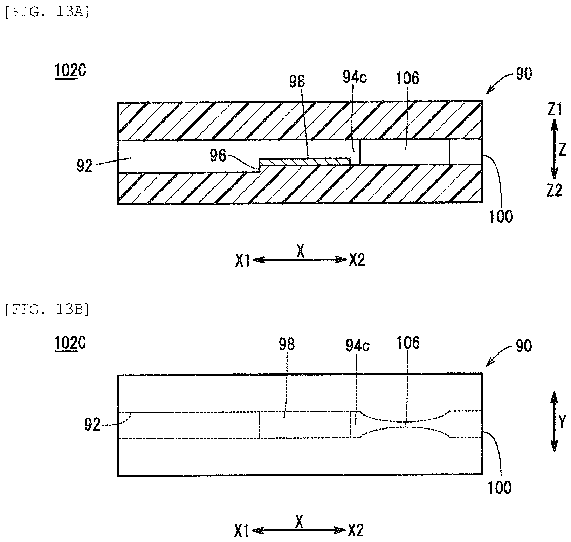

As illustrated in FIGS. 13A and 13B, in the test strip 102C according to Comparative Example 3, a buffer space 94c extends from a terminal end of the flow path 92 to an end surface of the main body portion 90 in the arrow X2 direction. In other words, the buffer space 94c is opened only on the end surface of the main body portion 90 in the arrow X2 direction. An end surface of the buffer space 94c in the arrow X2 direction functions as the vent hole 100. In FIG. 13B, the buffer space 94c is provided with a throttle portion 106 having a cross-sectional area smaller than a maximum cross-sectional area of the flow path 92. The throttle portion 106 is formed by causing inner surfaces of the buffer space 94c in the arrow Y direction to bulge inward.

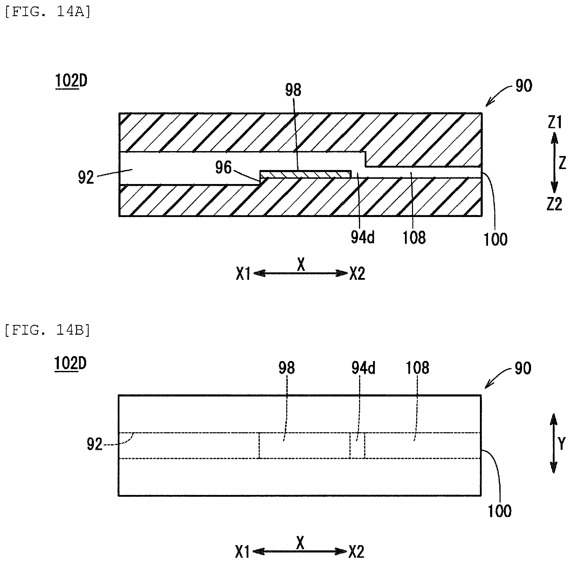

As illustrated in FIGS. 14A and 14B, in the test strip 102D according to Comparative Example 4, a buffer space 94d extends from a terminal end of the flow path 92 to an end surface of the main body portion 90 in an arrow X2 direction. In other words, the buffer space 94d is opened only on the end surface of the main body portion 90 in the arrow X2 direction. An opening portion at an end portion of the buffer space 94d in the X2 direction functions as the vent hole 100. In FIG. 14B, the buffer space 94d is provided with a throttle portion 108 having a cross-sectional area smaller than a maximum cross-sectional area of the flow path 92. The throttle portion 108 is a narrow flow path formed by causing inner surfaces of the buffer space 94d to bulge toward an inner surface direction of the flow path 92.

### [Test Method]

In Example 1 and Comparative Examples 1 to 4, a sample was spotted on the end surface of the main body portion 90 in the arrow X1 direction, and behaviors of the sample were checked by a CCD camera. As the sample, three types of liquids (RO water, an albumin aqueous solution, and blood) were used. As the RO water, RO water stained with nitro red (1 mg/ml) was used in order to facilitate visual recognition. As the albumin aqueous solution, 7 wt% of albumin was used. As the blood, blood having a hematocrit value (Ht) of 20 was used. The RO water imitates a sample that is most likely to leak from the main body portion 90. The albumin aqueous solution imitates blood plasma. A spotting amount of the sample was 5 µL. In Examples 1 and Comparative Examples 1 to 4, the volume in the flow path 92 is about 0.9 µL. An excess amount of sample was used, which was larger than an amount of specimen to be actually used. The test was carried out by fixing the main body portion 90 in a state where an intake port of the main body portion 90 was erected so as to face vertically upward, and imitating conditions that were harsher than that of an actual usage method.

### [Result]

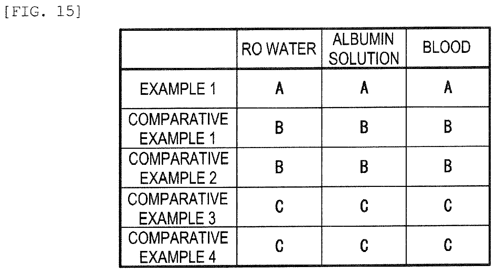

Test results of Example 1 and Comparative Examples 1 to 4 are illustrated in FIG. 15. As illustrated in FIG. 15, regarding evaluation of the tests, a case where no sample leaked from the vent hole 100 was indicated by A. A case where the sample did not leak outward from the vent hole 100 (the sample did not flow out of the vent hole 100) but bulge of a droplet of the sample due to interfacial tension was visually recognized in the vent hole 100 was indicated by B. A case where the sample leaked from the vent hole 100 was indicated by C.

In Example 1, the evaluation was A in all items of the RO water, the albumin liquid, and the blood. In contrast, in Comparative Examples 1 and 2, the evaluation was B in all items of the RO water, the albumin liquid, and the blood. In Comparative Examples 3 and 4, the evaluation was C in all items of the RO water, the albumin liquid, and the blood. In the cases which are evaluated as B, when the vent hole 100 was touched with a finger, the sample was attached to the finger.

As described above, a result was obtained that no sample leaked from the vent hole 100 when the cross-sectional area of the buffer space 94 is larger than a cross-sectional area of the flow path 92 in the connection portion between the buffer space 94 provided downstream of the reagent portion 98 and the flow path 92, and when a terminal cross section of the flow path 92 is connected to the buffer space 94 in either direction of the arrow Y direction and the arrow Z direction. That is, it is preferable that the buffer space 94 and the flow path 92 are connected in an extending direction (the arrow X direction) of the flow path 92, and the terminal cross section of the flow path 92 is spatially connected to a cross section of the buffer space 94. A result was obtained that the sample may leak when the terminal end of the flow path 26 has a shape that is largely opened at one surface in the Z direction (Comparative Examples 1 and 2), and the sample is likely to leak when a portion corresponding to the buffer space is narrower than the flow path 92 (Comparative Examples 3 and 4). From the above, it is shown that the blood can be effectively inhibited from leaking by providing the buffer space 94 having the characteristics described in Example 1 between the terminal end of the flow path 92 and the vent hole 100.

Next, a second test performed to check the effect of the invention will be described.

### [Sample]

Fifty test strips 12 manufactured by the above manufacturing method were prepared. In FIG. 8, in these test strips 12, a fluctuation of the displacement amount ΔD in the arrow X direction between the first buffer end 50 and the end surface 54 of the reagent piece 22 is set in a range of 0.02 mm to 0.26 mm.

### [Test Method]

In a state where the test strip 12 was fixed such that the intake portion 24 faced vertically upward, a sample was spotted on the intake portion 24, and presence or absence (leakage) of a sample from the flow path 26 into the buffer space 28 was checked with the CCD camera. As the sample, the above RO water was used. A spotting amount of the sample was 5 µL. A volume in the flow path 26 is about 0.9 µL.

### [Result]

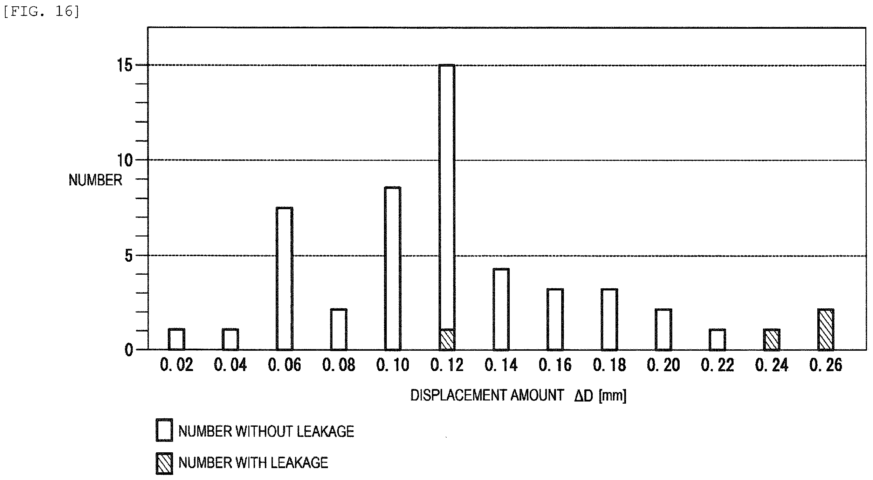

Test results of the fifty test strips 12 are illustrated in FIG. 16. FIG. 16 is a bar graph illustrating the number of test strips 12 in which the leakage of the sample from the flow path 26 to the buffer space 28 occurred (the number with leakage) and the number of test strips 12 in which the leakage of the sample from the flow path 26 to the buffer space 28 did not occur (the number without leakage). In FIG. 16, a horizontal axis indicates a magnitude of the displacement amount ΔD, and a vertical axis indicates the number of test strips 12. In the bar graph, the test strip in which the leakage of the sample from the flow path 26 into the buffer space 28 occurs is indicated by hatching. The displacement amount ΔD corresponds to a displacement length in the arrow X direction generated between an end of an upper surface of the flow path 26 and an end of a lower surface at the terminal end of the flow path 26. In the present embodiment, because the lower surface of the flow path 26 is the reagent piece 22 at the terminal end of the flow path 26 (a space connection portion between the flow path 26 and the buffer space 28), the displacement amount ΔD between an end of the reagent piece 22 on a buffer space 28 side and an end of the buffer space 28 on a flow path 26 side was evaluated.

As illustrated in FIG. 16, when the displacement amount ΔD was 0.24 mm or more, the sample leakage from the flow path 26 to the buffer space 28 was checked. In contrast, when the displacement amount ΔD was 0.22 mm or less, almost no sample leakage from the flow path 26 to the buffer space 28 occurred. When the displacement amount ΔD was 0.12 mm, the number of test strips in which sample leakage occurred was one, and the number of test strips in which no sample leakage occurred was fourteen. When the displacement amount ΔD was 0.10 mm or less, no sample leakage occurred in all the test strips 12.

As described above, in the test strips 12 in which the displacement amount ΔD (a maximum displacement amount) between the end of the reagent portion 22b (the reagent piece 22) on the buffer space 28 side and the end of the buffer space 28 on the flow path 26 side is 0.22 mm or less, the sample can be effectively inhibited from leaking from the flow path 26 to the buffer space 28. In the test strips 12 in which the displacement amount ΔD is less than 0.12 mm, the sample can be more effectively inhibited from leaking from the flow path 26 to the buffer space 28. In the test strips 12 in which the displacement amount ΔD is less than 0.10 mm, the sample can be still more effectively inhibited from leaking from the flow path 26 to the buffer space 28.

The invention is not limited to the embodiments described above, and various modifications can be made without departing from the gist of the invention.

The test strip 12 according to the invention is not limited to the application to the blood glucose level measurement system that measures the blood glucose level, and can be applied to various systems that optically measure an analyte component. For example, examples of the analyte to be measured in a medical site include a solution of a sample obtained from a living body such as urine (a ketone body or the like), interstitial fluid, or saliva in addition to the blood, and the analyte may be a stock solution or an experimental product subjected to a chemical treatment or the like. Alternatively, the component measurement system 10 can also be applied to a device that performs the component measurement of an analyte such as wastewater or an industrial sample.

The above embodiments are summarized as follows.

The above embodiments disclose a test strip (12, 12A) including: a flow path (26, 92) configured to be formed in a main body portion (20, 90); a reagent portion (22b, 98) configured to be provided in the flow path; and an intake portion (24) which is provided at a starting end of the flow path and through which a sample is introduced into the flow path, in which the main body portion is provided with a buffer space (28, 94) configured to communicate with a terminal end of the flow path, and a vent hole (30, 100) configured to be opened on an outer surface of the main body portion and configured to communicate with the buffer space, and in a region where the buffer space and the flow path are spatially connected, a cross-sectional area (Sb) of the buffer space is larger than a cross-sectional area (S) of the flow path.

In the above test strip, the buffer space may be formed in a rectangular parallelepiped shape.

In the above test strip, the flow path may be connected to the buffer space in a direction substantially perpendicular to the buffer space.

In the above test strip, the main body portion may be formed by stacking a plurality of film members.

In the above test strip, a maximum displacement amount (ΔD) between a terminal end of an upper surface of the flow path and a terminal end of a lower surface of the flow path in an arrangement direction of the flow path and the buffer space may be less than 0.24 mm.

In the above test strip, a maximum displacement amount between an end of the reagent portion on a buffer space side and an end of the buffer space on a flow path side in the arrangement direction of the flow path and the buffer space may be less than 0.24 mm.

## Claims

1. A test strip comprising:
a flow path formed in a main body portion;
a reagent portion provided in the flow path; and
an intake portion which is provided at a starting end of the flow path and through which a sample is introduced into the flow path, wherein
the main body portion is provided with
a buffer space communicating with a terminal end of the flow path, and
a vent hole opened at an outer surface of the main body portion and communicating with the buffer space, and
in a region where the buffer space and the flow path are spatially connected, a cross-sectional area of the buffer space is larger than a cross-sectional area of the flow path.

2. The test strip according to claim 1, wherein
the buffer space is formed in a rectangular parallelepiped shape.

3. The test strip according to claim 2, wherein
the flow path is connected to the buffer space in a direction substantially perpendicular to the buffer space.

4. The test strip according to any one of claims 1 to 3, wherein
the main body portion is formed by stacking a plurality of film members.

5. The test strip according to claim 4, wherein
a maximum displacement amount between a terminal end of an upper surface of the flow path and a terminal end of a lower surface of the flow path in an arrangement direction of the flow path and the buffer space is less than 0.24 mm.

6. The test strip according to claim 4, wherein
a maximum displacement amount between an end of the reagent portion on a buffer space side and an end of the buffer space on a flow path side in the arrangement direction of the flow path and the buffer space is less than 0.24 mm.
